# EUROPEAN PATENT APPLICATION

(11) **EP 4 427 722 A1**
(43) Date of publication of application: **11.09.2024**
(21) Application number: 24160794.4
(22) Date of filing: 01.03.2024
(51) Int. Cl.: A61K 6/64, A61K 6/71, A61K 6/887

(54) **MILL BLANK FOR DENTAL CUTTING AND MACHINING**

(30) Priority: 03.03.2023 JP 2023033258
(71) Applicant: Shofu Inc., Kyoto-shi, Kyoto 605-0983 (JP)
(72) Inventor: FUKAMI, Takahiro, Kyoto, 605-0983 (JP); KAWATA, Keita, Kyoto, 605-0983 (JP)
(74) Representative: Eisenführ Speiser

(57) **Abstract**

[Problem]

It has been difficult to prepare a mill blank for dental cutting and machining in a state of no cracks or breaks.

[Solution]

To provide a curable composition for use in a mill blank for dental cutting and machining, comprising
14 to 36 % by mass of (A) bifunctional urethane methacrylate monomer represented by Formula 1,
2 to 12 % by mass of (B) bifunctional methacrylate monomer not containing a urethane group,
60 to 80 % by mass of (C) inorganic filler,
0.1 to 0.5 % by mass of (D) thermal polymerization initiator, and
0.1 to 0.5 % % by mass of (E) chain transfer agent.

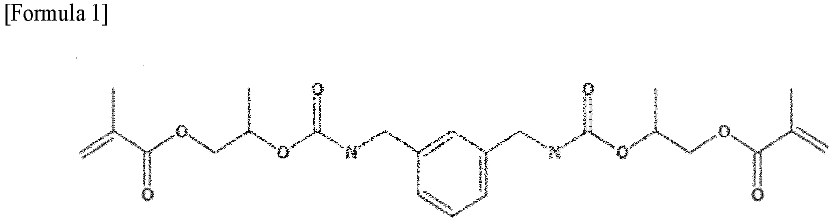

## Description

### BACKGROUND OF THE INVENTION

### Field of the Invention

The present invention relates to a mill blank for dental cutting and machining for preparing a dental prosthesis device (inlay, crown, bridge) that provide esthetic and functional restoration in cases where all or part of a crown portion of a natural tooth is missing, in the field of dental health care.

### Description of the Related Art

Recently, owing to the development of the dental CAD/CAM technique, the following technique has come to be used: a plaster model reproducing a lost tooth is optically scanned to produce a digital data of the lost tooth on a computer, a digital data of a prosthesis device is produced on the basis of the digital data of the lost tooth, and the prosthesis device is prepared by milling and machining a material on the basis of the digital data of the prosthesis device. For such a preparation of a prosthesis on the basis of the CAD/CAM technique, materials are required to be molded beforehand into a mill blank (blockshape, disc chape and the like). These mill blanks for dental cutting and machining are required to have high mechanical strength, color tone compatibility with a natural tooth, transparency equivalent to that of a natural tooth, and durability against deterioration of mechanical strength over time under severe oral environment.

### Relevant References

### Patent Literature

Patent Document 1: International Publication WO2015/152220 A1

Patent Document 1 discloses a dental material composition containing a urethane (meth)acrylate monomer. It is described that the mechanical strength can be further improved by controlling the ratio of acrylate groups to methacrylate groups within a certain range in the composition. However, it is difficult to prepare a mill blank for dental cutting and machining without crack or breakage by this technical content.

Conventionally, a mill blank for dental cutting and machining has been obtained by molding and curing a curable composition that could be applied to a mill blank for dental cutting and machining used in dental CAD/CAM technique, but it has been difficult to prepare a mill blank for dental cutting and machining without crack or breakage.

### SUMMARY OF THE INVENTION

### Technical Problem

In view of the above problems, an object of the present invention is to provide a curable composition for obtaining a mill blank for dental cutting and machining having high mechanical property without the presence of strain and crack and breakage, and a mill blank for dental cutting and machining which is a molded and cured product of this curable composition.

### Solution to Problem

As a result of intensive studies by the present inventors to solve the above problems, it has been found that, it is possible to prepare a mill blank for dental cutting and machining without crack or breakage by the curable composition of the present invention. The present invention provides a curable composition for use in mill blank for dental cutting and machining, comprising 14 to 36 % by mass of (A) bifunctional urethane methacrylate monomer represented by Formula 1, 2 to 12 % by mass of (B) bifunctional methacrylate monomer not containing a urethane group, 60 to 80 % by mass of (C) inorganic filler, 0.1 to 0.5 % by mass of (D) thermal polymerization initiator, and 0.1 to 0.5 % % by mass of (E) chain transfer agent.

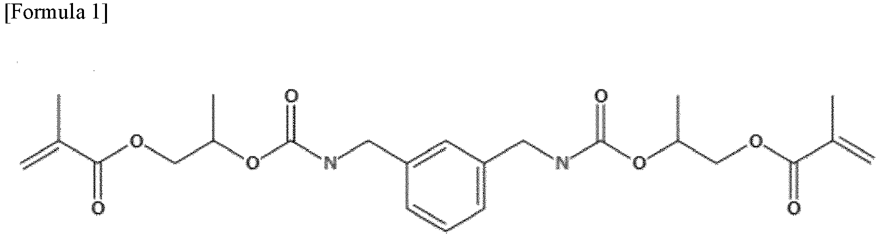

In the present invention, the mass ratio of the (A) bifunctional urethane methacrylate monomer represented by Formula 1 to the (B) bifunctional methacrylate monomer not containing a urethane group may be 90:10 to 70:30.

In the present invention, the (E) chain transfer agent may be a terpenoid compound.

The present invention provides a mill blank for dental cutting and machining which is a molded and cured product of the curable composition of the present invention.

### DETAILED DESCRIPTION OF THE PREFERRED EMBODIMENTS

### [(A) bifunctional urethane methacrylate monomer represented by Formula 1]

The (A) bifunctional urethane methacrylate monomer in the present invention is represented by following Formula 1.

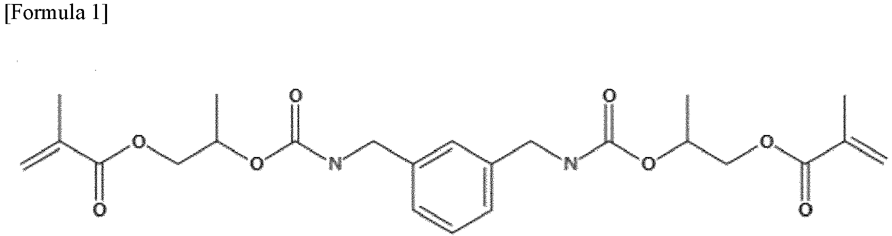

The content of the (A) bifunctional urethane methacrylate monomer represented by Formula 1 as a constituent component of the present invention is 14 to 36% by mass in the total amount of the curable composition, and may be 22 to 30% by mass in order to increase the mechanical strength in a state of no cracks or breaks. When the (A) bifunctional urethane methacrylate monomer represented by Formula 1 is less than 14% by mass, it becomes difficult to operate the curable composition. When the content of the (A) bifunctional urethane methacrylate monomer represented by Formula 1 exceeds 36% by mass, the mechanical strength of the mill blank for dental cutting and machining decreases.

### [(B) bifunctional methacrylate monomer not containing a urethane group]

Examples of the (B) bifunctional methacrylate monomer not containing a urethane group include an aliphatic bifunctional monomer and an aromatic bifunctional monomer.

Specific examples of an aliphatic bifunctional monomer include dimethacrylates such as ethylenediol, propylenediol, propanediol, butanediol, hexanediol, octanediol, nonanediol, decanediol, and eicosanediol; ethylene glycol dimethacrylate, diethylene glycol dimethacrylate, triethylene glycol dimethacrylate, propylene glycol dimethacrylate, tetraethylene glycol dimethacrylate, polyethylene glycol dimethacrylate, 1,3-butylene glycol dimethacrylate, neopentyl glycol dimethacrylate; 2-hydroxyethyl methacrylate, 2-hydroxypropyl methacrylate, 2-hydroxy-3-acryloyloxypropyl methacrylate, hydroxypivalic acid neopentyl glycol dimethacrylate, ethylene glycol dimethacrylate, diethylene glycol dimethacrylate, triethylene glycol dimethacrylate, butylene glycol dimethacrylate, neopentyl glycol dimethacrylate, propylene glycol dimethacrylate, polyethylene glycol dimethacrylate, 1,3-butanediol dimethacrylate, 1,4-butanediol dimethacrylate, 1,6-hexanediol dimethacrylate, and glycerol dimethacrylate.

Specific examples of an aromatic bifunctional monomer include 2,2-bis (4-methacryloyloxyphenyl) propane, 2,2-bis (4-(3-methacryloyloxy-2-hydroxypropoxy) phenyl) propane, 2,2-bis (4-methacryloyloxy ethoxyphenyl) propane, 2,2-bis (4-methacryloyloxy diethoxyphenyl) propane, 2,2-bis (4-methacryloyloxy tetraethoxyphenyl) propane, 2,2-bis (4-methacryloyloxy pentaethoxyphenyl) propane, 2,2-bis (4-methacryloyloxy dipropoxyphenyl) propane, 2-(4-methacryloyloxy ethoxyphenyl)-2-(4-methacryloyloxy diethoxyphenyl)propane, 2-(4-methacryloyloxy diethoxyphenyl)-2-(4-methacryloyloxy triethoxyphenyl)propane, 2-(4-methacryloyloxy dipropoxyphenyl)-2-(4-methacryloyloxy triethoxyphenyl)propane, 2,2-bis(4-methacryloyloxy dipropoxyphenyl)propane, and 2,2-bis(4-methacryloyloxy isopropoxyphenyl)propane.

In the present invention, the above described (B) bifunctional methacrylate monomer not containing a urethane group may be used alone or as a mixture of two or more.

The content of the (B) bifunctional methacrylate monomer not containing a urethane group as a constituent component of the present invention is 2 to 12% by mass in the total amount of the curable composition, and may be 4 to 9% by mass in order to increase the mechanical strength in a state of no cracks or breaks. When the (B) bifunctional methacrylate monomer not containing a urethane group is less than 2% by mass, it becomes difficult to operate the curable composition. When the content of the (B) bifunctional methacrylate monomer not containing a urethane group exceeds 12% by mass, the mechanical strength of the mill blank for dental cutting and machining decreases. As a specific example of the (B) bifunctional methacrylate monomer not containing a urethane group, an aliphatic difunctional monomer is preferable from the viewpoint of the operability of the curable composition.

The mass ratio of the (A) bifunctional urethane methacrylate monomer represented by Formula 1 to the (B) bifunctional methacrylate monomer not containing a urethane group is preferably 90:10 to 70:30, more preferably 85:15 to 75:25. When the mass ratio of the (A) bifunctional urethane methacrylate monomer represented by Formula 1 is larger than 90, there is a case where operation of the binder resin becomes difficult and it is not possible to mold a mill blank for dental cutting and machining in a uniform state. On the other hand, when the mass ratio of the (A) bifunctional urethane methacrylate monomer represented by Formula 1 is less than 70, there is a case where the mechanical strength of the mill blank for dental cutting and machining decreases due to the polymer structure.

The curable composition of the present invention may contain a polymerization component other than the bifunctional urethane methacrylate monomer, to the extent that the effects of the present invention are not impaired.

The curable composition of the present invention may not contain any monomer component other than the difunctional acrylate monomer. The curable composition of the present invention may not contain any monomer component other than the (A) bifunctional urethane methacrylate monomer represented by Formula 1 and the (B) bifunctional methacrylate monomer not containing a urethane group.

### [(C) inorganic filler]

For the (C) inorganic filler as a constituent component of the present invention, heretofore known inorganic fillers used for dental materials can be used without any limitation. Examples of the material of the inorganic filler include: kaolin, talc, quartz, silica, colloidal silica, alumina, aluminosilicate, silicon nitride, barium sulfate, calcium phosphate, various glasses (such as fluoro-glass, borosilicate glass, soda glass, barium glass, glass containing strontium or zirconium, glass ceramics, fluoroaluminosilicate glass and synthetic glass by sol-gel method), zirconia, zirconium silicate and hydroxy apatite. The shape, the particle size distribution, the average particle diameter and the like of the inorganic filler are not particularly limited. However, similarly to the common dental resin material (such as composite resin) compounded with an inorganic filler, while the material strength is being enhanced, the material strength and the surface lubricative property are required to be made compatible with each other. Accordingly, the average particle diameter is desired to be 0.01 to 50 µm, preferably 0.01 to 10 µm, and further preferably 0.01 to 1 µm. In the present invention, the average particle diameter means a particle diameter at which the cumulative value of the particle diameter is 50% and such average particle diameter can be measured by, for example, a laser diffraction method.

In addition, a curable composition compounded with an inorganic filler having an average particle diameter of 0.1 µm or less is one of the preferable embodiment. As specific examples of material for such inorganic fillers having a small particle diameter, silica, alumina, zirconia, and titania are preferable. The compound of such an inorganic filler having a small particle diameter is advantageous in obtaining surface lubricative property for a mill blank for dental cutting and machining. The curable composition of the present invention may contain no fillers other than the inorganic filler having an average particle diameter of 0.1 µm or less.

As the (C) inorganic filler, which is one of the constituent components, it is possible to compound a porous inorganic filler. The porous inorganic filler refers to an inorganic filler having at least one or more pores. The presence or absence of pores in an inorganic filler can be measured, for example, by gas adsorption method or mercury intrusion method. More specifically, it refers to an inorganic filler whose pore volume measured by gas adsorption method is 0.01 cc/g or more.

The (C) inorganic filler used in the present invention is preferably surface-treated. Examples of the surface treatment material include silane compounds such as vinyltrimethoxysilane, vinyltriethoxysilane, vinyltrichlorosilane, vinyltri (β-methoxyethoxy) silane, γ-methacryloxypropyl trimethoxysilane, γ-glycidoxypropyl trimethoxysilane, γ-mercaptopropyl trimethoxysilane and γ-aminopropyl triethoxysilane.

The (C) inorganic filler used in the present invention may be used alone or in combination of two or more types as appropriate. The compounding of the inorganic filler may be appropriately determined taking into consideration the operability (viscosity) of the curable composition, which is the precursor of the mill blank for dental cutting and machining, and the mechanical property of the mill blank for dental cutting and machining.

For the (C) inorganic filler as a constituent component of the present invention, it is essential requirement that the content is 60 to 80% by mass. In the prosthesis device obtained from the mill blank for dental cutting and machining according to the present invention, it is basically intended to function over a long term (2 years or more) in the oral cavity. Accordingly, the durability under the high temperature and the high humidity specific to the inside of the oral cavity is required, and it is required to reinforce with a certain amount or more of an inorganic filler in order to achieve this. When the compounding ratio of the inorganic filler content is less than 60% by mass, it is difficult to make the material function stably for a long period of time. When the compounding ratio of the inorganic filler content exceeds 80% by mass, it becomes difficult to operate the curable composition. The curable composition of the present invention may contain 65 to 70% by mass of the (C) inorganic filler. The curable composition of the present invention may contain no fillers other than the (C) inorganic filler.

### [(D) thermal polymerization initiator]

For the (D) thermal polymerization initiator as a constituent component of the present invention, heretofore known thermal polymerization initiator used for dental materials can be used without any limitation. For example, diacyl peroxides, peroxy esters, dialkyl peroxides, peroxyketals, ketone peroxides and hydroperoxides are effective. Specific examples of the diacyl peroxides include benzoylperoxide, 2,4-dichlorobenzoylperoxide and m-toluoyl peroxide.

The content of the above described (D) thermal polymerization initiator is 0.1 to 0.5% by mass in the total amount of the curable composition. When the content of the (D) thermal polymerization initiator is too small, an insufficient polymerization of the polymerizable monomer occurs. In contrast, when the compounding amount of the (D) thermal polymerization initiator is too large, the termination reaction during polymerization is accelerated and consequently the strength degradation of the mill blank for dental cutting and machining is caused.

In addition to the above described (D) thermal polymerization initiator, heretofore known polymerization accelerators may also be used without any limitation. Examples include N,N-dimethylaniline, N,N-diethylaniline, N,N-dibenzylaniline, N,N-dimethyl-p-toluidine, N,N-diethyl-p-toluidine, N,N-dihydroxyethyl-p-toluidine, N,N-dimethylbenzoic acid, N,N-diethylbenzoic acid, N,N-dimethylbenzoic acid ethyl, N,N-diethylbenzoic acid ethyl, N,N-dimethylbenzoic acid methyl, N,N-diethylbenzoic acid methyl, N,N-dimethylaminobenzaldehyde, N,N-dihydroxyethylaniline, p-dimethylaminophenethyl alcohol, N,N-dimethylaminoethyl methacrylate, N,N-dimethylaminomethyl methacrylate, triethylamine, tributylamine, tripropylamine, and N-ethylethanolamine.

When a polymerization accelerator is compounded, the content is 0.1 to 0.5 by mass in the total amount of the curable composition. The curable composition of the present invention may contain a photopolymerization initiator and/or a chemical polymerization initiator in addition to the (D) thermal polymerization initiator. The curable composition of the present invention may not contain a photopolymerization initiator and a chemical polymerization initiator. The curable composition of the present invention may not contain a polymerization accelerator.

### [(E) chain transfer agent]

For the (E) chain transfer agent as a constituent component of the present invention, any terpenoid compound used for dental materials can be used without being particular limited. Specific examples include α-terpinene, β-terpinene, and γ-terpinene.

The content of the above described (E) chain transfer agent is 0.1 to 0.5% by mass in the total amount of the entire curable composition. In the curable composition of the present invention, it is possiblet to uniformly porimerize and cure by compounding a chain transfer agent in the polymerizable component.

In the curable composition of the present invention, for the colorant as a constituent component, the heretofore known colorants used in the dental materials are used without any limitation. As the colorants, either an inorganic compound-based colorant or an organic compound-based colorant can be used. In particular, a white colorant is useful for adjusting the transparency of each of the layers in the present invention.

In the curable composition of the present invention, heretofore known various additives can be mixed if necessary. Examples of such additives include a polymerization inhibitor, a discoloration preventing agent, a fluorescent agent, an ultraviolet absorber and an antibacterial agent.

### [Example]

Hereinafter, the present invention is described by way of Examples in more detail, and specifically, but the present invention is not limited to these Examples.

### (Bending strength test)

### Purpose: Evaluation of bending strength of mill blank for dental cutting and machining

Method: Plates of (1.2 ± 0.2) × (4.0 ± 0.2) × 14 mm or more were cut out from the prototype mill blank for dental cutting and machining, and the surface was polished with #2000 waterproof abrasive paper. These were immersed in distilled water in a sealable container and stored at 37 °C for 7 days to be used as test specimens. Bending strength was measured using an Instron universal testing machine (Instron 5567: manufactured by Instron) at a distance between supporting points of 12 mm and a crosshead speed of 1 mm/min.

Evaluation criteria were as follows.
AA: 230 MPa or more
A: 200 MPa or more and less than 230 MPa
B: 160 MPa or more and less than 200 MPa
C: less than 160 Mpa

### (Evaluation of cracks and breaks in molded bodies)

Purpose: Evaluation of cracks in mill blank for dental cutting and machining
Method: Cracks and breaks were evaluated by visually confirming 10 molded mill blank for dental cutting and machining.

Evaluation criteria were as follows.
A: no cracks
B: crack occurrence rate less than 30%
C: crack occurrence rate 30% or more

### (Consistency (flow value))

Purpose: Evaluation of the consistency (flow value) of the curable composition which is a precursor of a mill blank for dental cutting and machining.
Method: the curable composition after kneading was filled into a cylindrical tube (inner diameter 7.5 mm). The curable composition was gently extruded onto the center of a glass plate (70 × 70 × 1 mm), and a glass plate of the same type was placed on top. A load (400 gf) was gently applied to the center thereof, and the dimensions of the curable composition were confirmed after 180 seconds from applying the load.

Evaluation criteria were as follows.
A: 20 to 40 mm,
B: 10 to less than 20 mm or more than 40 to 50 mm
C: more than 50 mm or less than 10 mm

### [Bifunctional urethane methacrylate monomer]

### Bifunctional urethane methacrylate monomer represented by Formula 1 (Monomer (A))

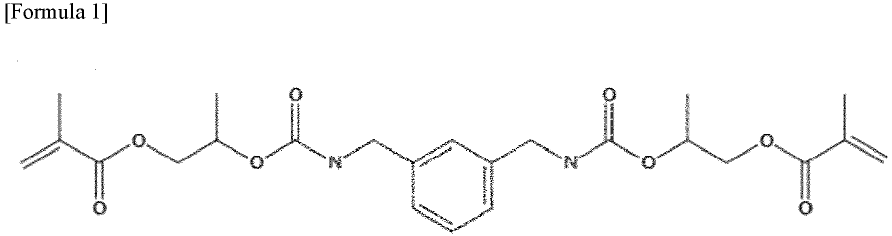

### Bifunctional urethane methacrylate monomer other than (A) (Monomer (A'))

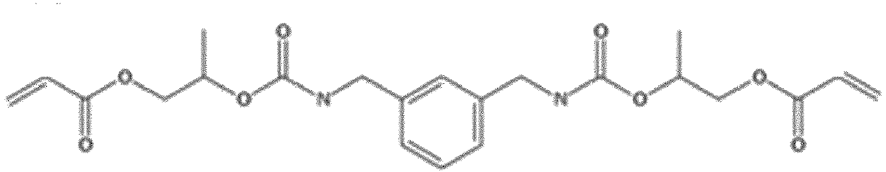

### [(B) bifunctional methacrylate monomer not containing a urethane group]

TEGDMA: triethylene glycol methacrylate (manufactured by SHIN-NAKAMURA CHEMICAL CO, LTD.)
701: 2-hydroxy-1,3-dimethacryloxypropane (manufactured by SHIN-NAKAMURA CHEMICAL CO, LTD.)

### [Raw materials for (C) inorganic filler]

UF1.5: Strontium glass (average primary particle diameter: 1.5 µm, manufactured by Schott)
UF1.0: Strontium glass (average primary particle diameter: 1.0 µm, manufactured by Schott)
UF0.4-S: Strontium glass (average primary particle diameter: 0.4 µm, manufactured by Schott)
UF0.4-B: Barium glass (average primary particle diameter: 0.4 µm, manufactured by Schott)
OX-50: Fine silica particles (average primary particle diameter: 0.04 µm, manufactured by Nippon Aerosil)

### [Surface treatment agent for (C) inorganic filler]

γ-MPS: γ-methacryloxypropyl trimethoxysilane (manufactured by Shin-Etsu Chemical Co., Ltd.)

### [(D) thermal polymerization initiator]

BPO: benzoyl peroxide (Wako Pure Chemical Industries, Ltd.)
V-601: dimethyl-2,2'-azobis(2-methylpropionate) (Wako Pure Chemical Industries, Ltd.)

### [(E) chain transfer agent]

α-terpinene: p-Mentha-1,3-diene, 1-isopropyl-4-methyl-1,3-cyclohexadiene (Tokyo Chemical Industry Co., Ltd.)
β-terpinene: p-Mentha-1,4-diene, 1-isopropyl-4-methyl-1,4-cyclohexadiene (Tokyo Chemical Industry Co., Ltd.)

### [(C) inorganic filler]

The above discribed raw materials for (C) inorganic filler and surface treatment agent were used to produce Inorganic fillers (C-1) to (C-5) according to following Inorganic filler production examples 1 to 5.

### [Inorganic filler production example 1]

UF1.5 (average primary particle diamter 1.5 µm, manufactured by Schott) in 100 parts by mass was mixed with 3 parts by mass of γ-MPS, 10 parts by mass of ethanol, 2 parts by mass of distilled water, and 0.1 parts by mass of phosphoric acid. In addition, the surface was treated by drying at 100 °C for 3 hours to produce Inorganic filler (C-1).

### [Inorganic filler production example 2]

UF1.0 (average primary particle diamter 1.0 µm, manufactured by Schott) in 100 parts by mass was mixed with 4 parts by mass of γ-MPS, 10 parts by mass of ethanol, 2 parts by mass of distilled water, and 0.1 parts by mass of phosphoric acid. In addition, the surface was treated by drying at 100 °C for 3 hours to produce Inorganic filler (C-2).

### [Inorganic filler production example 3]

UF0.4-S (strontium glass, average primary particle diamter 0.4 µm, manufactured by Schott) in 100 parts by mass was mixed with 9 parts by mass of γ-MPS, 10 parts by mass of ethanol, 2 parts by mass of distilled water, and 0.1 parts by mass of phosphoric acid. In addition, the surface was treated by drying at 100 °C for 3 hours to produce Inorganic filler (C-3).

### [Inorganic filler production example 4]

UF0.4-B (barium glass, average primary particle diamter 0.4 µm, manufactured by Schott) in 100 parts by mass was mixed with 9 parts by mass of γ-MPS, 10 parts by mass of ethanol, 2 parts by mass of distilled water, and 0.1 parts by mass of phosphoric acid. In addition, the surface was treated by drying at 100 °C for 3 hours to produce Inorganic filler (C-4).

### [Inorganic filler production example 5]

OX-50 (average primary particle diamter 0.04 µm, manufactured by Nippon Aerosil) in 100 parts by mass was mixed with 10 parts by mass of γ-MPS, 10 parts by mass of ethanol, 2 parts by mass of distilled water, and 0.1 parts by mass of phosphoric acid. In addition, the surface was treated by drying at 100 °C for 3 hours to produce Inorganic filler (C-5).

### (Preparation of curable composition)

The components shown in Tables 1 to 3 were mixed and defoamed under reduced pressure to prepare a curable composition. The content of each component is expressed by percentage by mass.

### (Mill blank for dental cutting and machining)

The curable composition was filled into a 14.5 mm × 14.5 mm × 18.0 mm block preparation mold. The mold was heated to 110 °C, and the curable composition was polymerized and cured to prepare a mill blank for dental cutting and machining.

Mill blanks for dental cutting and machining (Examples 1 to 41 and Comparative Examples 1 to 7) were prepared using the curable compositions shown in Tables 1 to 3. Tables 1 to 3 also show the mass % of each component, the presence or absence of cracks, and the measured bending strength and flow value of the curable composition for each Example or Comparative Example.

**[Table 1]**

| | | Example 1 | Example 2 | Example 3 | Example 4 | Example 5 | Example 6 | Example 7 | Example 8 | Example 9 |
|---|---|---|---|---|---|---|---|---|---|---|
| Monomer (A) | | 18.0 | 14.0 | 35.6 | 28.0 | 18.0 | 14.0 | 35.8 | 28.0 | 18.0 |
| Monomer (A') | | | | | | | | | | |
| (Monomer (B) | TEGDMA | 2.0 | 6.0 | 4.0 | 11.6 | 2.0 | 6.0 | 4.0 | 11.8 | 2.0 |
| | 701 | | | | | | | | | |
| Ratio of monomer (A) to monomer (B) | | 90.0 | 70.0 | 89.9 | 70.7 | 90.0 | 70.0 | 89.9 | 70.4 | 90.0 |
| (C) inorganic filler | C-1 | | | | | | | | | |
| | C-2 | | | | | | | | | |
| | C-3 | 78.6 | 78.6 | 59.0 | 59.0 | 78.8 | 78.8 | 59.0 | 59.0 | 78.0 |
| | C-4 | | | | | | | | | |
| | C-5 | 1.0 | 1.0 | 1.0 | 1.0 | 1.0 | 1.0 | 1.0 | 1.0 | 1.0 |
| Total of (C) inorganic filler | | 79.6 | 79.6 | 60.0 | 60.0 | 79.8 | 79.8 | 60.0 | 60.0 | 79.0 |
| (D) thermal polymerization initiator | BPO | 0.2 | 0.2 | 0.2 | 0.2 | 0.1 | 0.1 | 0.1 | 0.1 | 0.5 |
| | V-601 | | | | | | | | | |
| (E) chain transfer agent | α·terpinene | 0.2 | 0.2 | 0.2 | 0.2 | 0.1 | 0.1 | 0.1 | 0.1 | 0.5 |
| | β-terpinene | | | | | | | | | |
| Bending strength (Mpa) | | 226 | 211 | 194 | 180 | 204 | 192 | 173 | 165 | 228 |
| | | A | A | B | B | A | B | B | B | A |
| Cracks | | 2 | 1 | 0 | 0 | 1 | 0 | 0 | 0 | 2 |
| | | B | B | A | A | B | A | A | A | B |
| Flow value | | 11 | 15 | 33 | 35 | 12 | 15 | 33 | 36 | 13 |
| | | B | B | A | A | B | B | A | A | B |

| | | Example 10 | Example 11 | Example 12 | Example 13 | Example 14 | Example 15 | Example 16 | Example 17 | Example 18 |
|---|---|---|---|---|---|---|---|---|---|---|
| Monomer (A) | | 14.0 | 35.0 | 28.0 | 180 | 140 | 35.6 | 28.0 | 180 | 140 |
| Monomer (A') | | | | | | | | | | |
| (Monomer (B) | TEGDMA | 6.0 | 4.0 | 11.0 | | | | | 2.0 | 6.0 |
| | 701 | | | | 2.0 | 6.0 | 4.0 | 11.6 | | |
| Ratio of monomer (A) to monomer (B) | | 70.0 | 89.7 | 71.8 | 90.0 | 70.0 | 89.9 | 70.7 | 90.0 | 70.0 |
| (C) inorganic filler | C-1 | | | | | | | | | |
| | C-2 | | | | | | | | | |
| | C-3 | 78.0 | 59.0 | 59.0 | 78.6 | 78.6 | 59.0 | 59.0 | 78.6 | 78.6 |
| | C-4 | | | | | | | | | |
| | C-5 | 1.0 | 1.0 | 1.0 | 1.0 | 1.0 | 1.0 | 1.0 | 1.0 | 1.0 |
| Total of (C) inorganic filler | | 79.0 | 60.0 | 60.0 | 79.6 | 79.6 | 60.0 | 60.0 | 79.6 | 79.6 |
| (D) thermal nolvmerization initiator | BPO | 0.5 | 05 | 0.5 | 0.2 | 0.2 | 0.2 | 0.2 | | |
| | V-601 | | | | | | | | 0.2 | 0.2 |
| (E) chain transfer agent | α-terpinene | 0.5 | 0.5 | 0.5 | 0.2 | 0.2 | 0.2 | 0.2 | 0.2 | 0.2 |
| | β-terpinene | | | | | | | | | |
| Bending strength (Mpa) | | 209 | 190 | 176 | 226 | 212 | 193 | 172 | 224 | 220 |
| | | A | B | B | A | A | B | B | A | A |
| Cracks | | 1 | 0 | 0 | 1 | 1 | 0 | 0 | 0 | 0 |
| | | B | A | A | B | B | A | A | A | A |
| Flow value | | 16 | 32 | 34 | 12 | 17 | 33 | 37 | 14 | 16 |
| | | B | A | A | B | B | A | A | B | B |

**[Table 2]**

| | | Example 19 | Example 20 | Examnple 21 | Example 22 | Example 23 | Example 24 | Example 25 | Example 26 | Example 27 |
|---|---|---|---|---|---|---|---|---|---|---|
| Monomer (A) | | 35.6 | 28.0 | 18.0 | 14.0 | 35.6 | 28.0 | 18.0 | 14.0 | 35.6 |
| Monomer (A') | | | | | | | | | | |
| (Monomer (B) | TEGDMA | 4.0 | 11.6 | 2.0 | 6.0 | 4.0 | 11.6 | 2.0 | 6.0 | 4.0 |
| | 701 | | | | | | | | | |
| Ratio of monomer (A) to monomer (B) | | 89.9 | 70.7 | 90.0 | 70.0 | 89.9 | 70.7 | 90.0 | 70.0 | 89.9 |
| (C) inorganic filler | C-1 | | | | | | | 78.6 | 78.6 | 59.0 |
| | C-2 | | | | | | | | | |
| | C-3 | 59.0 | 59.0 | 78.6 | 78.6 | 59.0 | 59.0 | | | |
| | C-4 | | | | | | | | | |
| | C-5 | 1.0 | 1.0 | 1.0 | 1.0 | 1.0 | 1.0 | 1.0 | 1.0 | 1.0 |
| Total of (C) inorganic filler | | 60.0 | 60.0 | 79.6 | 79.6 | 60.0 | 60.0 | 79.6 | 79.6 | 60.0 |
| (D) thermal polymerization initiator | BPO | | | 0.2 | 0.2 | 0.2 | 0.2 | 0.2 | 0.2 | 0.2 |
| | V-601 | 0.2 | 0.2 | | | | | | | |
| (E) chain transfer agent | α-terpinene | 0.2 | 0.2 | | | | | 0.2 | 0.2 | 0.2 |
| | β-terpinene | | | 0.2 | 0.2 | 0.2 | 0.2 | | | |
| Bending (Mpa) | | 188 | 167 | 231 | 215 | 196 | 175 | 233 | 213 | 185 |
| | | B | B | AA | A | B | B | AA | A | B |
| strength Cracks | | 0 | 0 | 1 | 2 | 0 | 0 | 1 | 0 | 0 |
| | | A | A | B | B | A | A | B | B | A |
| Flow value | | 31 | 38 | 12 | 15 | 32 | 37 | 24 | 27 | 38 |
| | | A | A | B | B | A | A | A | A | A |

| | | Example 28 | Example 29 | Example 30 | Example 31 | Example 32 | Example 33 | Example 34 | Example 35 | Example 36 |
|---|---|---|---|---|---|---|---|---|---|---|
| Monomer (A) | | 28.0 | 18.0 | 14.0 | 35.6 | 28.0 | 18.0 | 14.0 | 35.6 | 28.0 |
| Monomer (A') | | | | | | | | | | |
| (Monomer (B) | TEGDMA | 11.6 | 2.0 | 6.0 | 4.0 | 11.6 | 2.0 | 6.0 | 4.0 | 11.6 |
| | 701 | | | | | | | | | |
| Ratio of monomer (A) to monomer (B) | | 70.7 | 90.0 | 70.0 | 89.9 | 70.7 | 90.0 | 70.0 | 89.9 | 70.7 |
| (C) inorganic filler | C-1 | 59.0 | | | | | | | | |
| | C-2 | | 78.6 | 78.6 | 59.0 | 59.0 | | | | |
| | C-3 | | | | | | | | | |
| | C-4 | | | | | | 78.6 | 78.6 | 59.0 | 59.0 |
| | C-5 | 1.0 | 1.0 | 1.0 | 1.0 | 1.0 | 1.0 | 1.0 | 1.0 | 1.0 |
| Total of (C) inorganic filler | | 60.0 | 79.6 | 79.6 | 60.0 | 60.0 | 79.6 | 79.6 | 60.0 | 60.0 |
| (D) thermal nolvmerization initiator | BPO | 0.2 | 0.2 | 0.2 | 0.2 | 0.2 | 0.2 | 0.2 | 0.2 | 0.2 |
| | V·601 | | | | | | | | | |
| (E) chain transfer agent | α·terpinene | 0.2 | 0.2 | 0.2 | 0.2 | 0.2 | 0.2 | 0.2 | 0.2 | 0.2 |
| | β·terpinene | | | | | | | | | |
| Bending strength (Mpa) | | 168 | 228 | 214 | 193 | 172 | 233 | 213 | 201 | 188 |
| | | B | A | A | B | B | AA | A | A | B |
| Cracks | | 0 | 2 | 1 | 0 | 0 | 1 | 1 | 0 | 0 |
| | | A | B | B | A | A | B | B | A | A |
| Flow value | | 43 | 23 | 25 | 37 | 39 | 13 | 17 | 32 | 37 |
| | | B | A | A | A | A | B | B | A | A |

**[Table 3]**

| | | Example 37 | Example 38 | Example 39 | Examnle 40 | Examnle 41 | Example 42 |
|---|---|---|---|---|---|---|---|
| Monomer (A) | | 25.5 | 22.5 | 29.4 | 26.0 | 27.6 | 27.6 |
| Monomer (A') | | | | | | | |
| (Monomer (B) | TEGDMA | 4.5 | 7.5 | 5.2 | 8.6 | 7.0 | 3.5 |
| | 701 | | | | | | 3.5 |
| Ratio of monomer (A) to monomer (B) | | 85.0 | 75.0 | 85.0 | 75.1 | 79.8 | 79.8 |
| (C) inorganic filler | C-1 | | | | | | |
| | C-2 | | | | | | |
| | C-3 | 68.6 | 68.6 | 64.0 | 64.0 | 64.0 | 64.0 |
| | C-4 | | | | | | |
| | C-5 | 1.0 | 1.0 | 1.0 | 1.0 | 1.0 | 1.0 |
| Total of (C) inorganic filler | | 69.6 | 69.6 | 65.0 | 65.0 | 65.0 | 65.0 |
| (D) thermal polymerization initiator | BPO | 0.2 | 0.2 | 0.2 | 0.2 | 0.2 | 0.20 |
| | V-601 | | | | | | |
| (E) chain transfer agent | α-terpinene | 0.2 | 0.2 | 0.2 | 0.2 | 0.2 | 0.20 |
| | β-terpinene | | | | | | |
| Bending strength (MpaJ | | 246 | 238 | 236 | 231 | 241 | 237 |
| | | AA | AA | AA | AA | AA | AA |
| Cracks | | 0 | 0 | 0 | 0 | 0 | 0 |
| | | A | A | A | A | A | A |
| Flow value | | 23 | 25 | 28 | 32 | 29 | 31 |
| | | A | A | A | A | A | A |

| | | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 |
|---|---|---|---|---|---|---|---|---|---|---|
| Monomer (A) | | 13.5 | 10.5 | 40.5 | 31.5 | 32.9 | 20.8 | | 27.9 | 26.0 |
| Monomer (A') | | | | | | | | 27.6 | | |
| (Monomer (B) | TEGDMA | 1.5 | 4.5 | 4.5 | 13.5 | 1.7 | 13.8 | 7.0 | 7.0 | 7.0 |
| | 701 | | | | | | | | | |
| Ratio of monomer (A) to monomer (B) | | 90.0 | 70.0 | 90.0 | 70.0 | 95.1 | 60.1 | 79.8 | 79.9 | 78.8 |
| (C) inorganic filler | C-1 | | | | | | | | | |
| | C-2 | | | | | | | | | |
| | C-3 | 83.6 | 83.6 | 53.6 | 53.6 | 64.0 | 64.0 | 64.0 | 64.0 | 64.0 |
| | C-4 | | | | | | | | | |
| | C-5 | 1.0 | 1.0 | 1.0 | 1.0 | 1.0 | 1.0 | 1.0 | 1.0 | 1.0 |
| Total of (C) inorganic filler | | 84.6 | 84.6 | 54.6 | 54.6 | 65.0 | 65.0 | 65.0 | 65.0 | 65.0 |
| (D) thermal nolvmerization initiator | BPO | 0.2 | 0.2 | 0.2 | 0.2 | 0.2 | 0.2 | 0.2 | 0.05 | 1.00 |
| | V-601 | | | | | | | | | |
| (E) chain transfer agent | α-terpinene | 0.2 | 0.2 | 0.2 | 0.2 | 0.2 | 0.2 | 0.2 | 0.05 | 1.00 |
| | β-terpinene | | | | | | | | | |
| Bending (Mpa) | | 132 | 135 | 148 | 139 | 185 | 159 | 155 | 158 | 214 |
| | | C | C | C | C | B | C | C | C | A |
| strength Cracks | | 8 | 6 | 0 | 0 | 7 | 0 | 4 | 0 | 4 |
| | | C | C | A | A | C | A | C | A | C |
| Flow value | | 8 | 9 | 51 | 55 | 23 | 43 | 30 | 31 | 30 |
| | | C | C | C | C | A | B | A | A | A |

From the above results, the curable composition for use in a mill blank for dental cutting and machining of the present invention had high mechanical property without the presence of distortion, cracks, or chipping. This is believed to be due to the appropriate specification of each component of the curable composition. Therefore, the curable composition for use in mill blank for dental cutting and machining of the present invention has dramatically improved manufacturability and mechanical properties compared to conventional.

With respect to the use of substantially any plural and/or singular terms herein, those having skill in the art can translate from the plural to the singular and/or from the singular to the plural as is appropriate to the context.

Although the description herein has been given with reference to the drawings and embodiments, it should be noted that those skilled in the art may make various changes and modifications on the basis of this invention without difficulty. Accordingly, any such changes and modifications are intended to be included in the scope of the embodiments.

### [Industrial applicability]

The present invention relates to a mill blank for dental cutting and machining for preparing a dental prosthesis device (inlay, crown, bridge) that provide esthetic and functional restoration in cases where all or part of a crown portion of a natural tooth is missing, in the field of dental health care. It is possible to prepare a mill blank for dental cutting and machining without crack or breakage by the curable composition of the present invention.

## Claims

1. A curable composition for use in a mill blank for dental cutting and machining, comprising
14 to 36 % by mass of (A) bifunctional urethane methacrylate monomer represented by Formula 1,
2 to 12 % by mass of (B) bifunctional methacrylate monomer not containing a urethane group,
60 to 80 % by mass of (C) inorganic filler,
0.1 to 0.5 % by mass of (D) thermal polymerization initiator, and
0.1 to 0.5 % % by mass of (E) chain transfer agent.

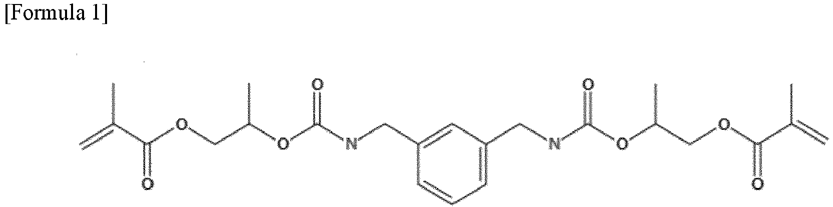

2. The curable composition for use in a mill blank for dental cutting and machining according to claim 1, wherein
the mass ratio of the (A) bifunctional urethane methacrylate monomer represented by Formula 1 to the (B) bifunctional methacrylate monomer not containing a urethane group is 90:10 to 70:30.

3. The curable composition for use in a mill blank for dental cutting and machining according to claim 1 or 2, wherein
the (E) chain transfer agent is a terpenoid compound.

4. The curable composition for use in a mill blank for dental cutting and machining according to any one of claims 1 to 3, wherein
the curable composition for use in a mill blank for dental cutting and machining does not contain any monomer component other than the (A) bifunctional urethane methacrylate monomer represented by Formula 1 and the (B) bifunctional methacrylate monomer not containing a urethane group.

5. A mill blank for dental cutting and machining, wherein
the mill blank for dental cutting and machining is a molded and cured product of the curable composition according to any one of claims 1 to 4.
